# EUROPEAN PATENT SPECIFICATION

(11) **EP 2 786 141 B1**
(45) Date of publication and mention of the grant of the patent: **18.05.2016**
(21) Application number: 12853020.1
(22) Date of filing: 29.11.2012
(51) Int. Cl.: G01N 33/50, G01N 33/52, G01N 33/53

(54) **DEVICE WITH INTEGRATED ALLERGY TESTING**
VORRICHTUNG MIT INTEGRIERTEM ALLERGIETEST
DISPOSITIF AYANT UN TEST ALLERGIQUE INTÉGRÉ

(30) Priority: 29.11.2011 US 201161564593 P
(43) Date of publication of application: 08.10.2014
(73) Proprietor: Teleflex Medical Incorporated, Research Triangle Park, NC 27709 (US)
(72) Inventor: GUPTA, Nisha, Audubon Pennsylvania 19403 (US); ROWE, David, Sinking Spring Pennsylvania 19408 (US); DENLINGER, Rodney, Lancaster Pennsylvania 19402 (US)
(74) Representative: Cordina, Kevin John
(86) International application number: PCT/US2012/067095
(87) International publication number: WO 2013/082301

(56) References cited:
- US-A- 5 368 029
- US-A1- 2003 109 777
- US-A1- 2003 150 745
- US-A1- 2004 116 830
- US-A1- 2004 210 247
- US-A1- 2007 161 926
- US-A1- 2008 255 473
- US-A1- 2009 112 125
- US-A1- 2011 046 453

## Description

### Field of the disclosure

The present disclosure generally relates to a medical testing device. More particularly, the present disclosure pertains to a device having an integrated allergy testing system.

### Background of the disclosure

During surgical procedures and other medical procedures, it is often necessary to determine if a patient has any allergies that may be aggravated during the procedure. Testing for allergies can be a long process. Often, an immediate answer is needed during the procedure.

Extensive use of certain compounds in home and health care products (oral care products, contact lens care products, dressings, ointments, soaps, cosmetics) is causing a larger population to become pre-sensitized to such particular agents. When a pre-sensitized person comes in contact with such an agent, there is a high probability that person could develop much stronger symptoms as severe as anaphylactic shock with cardiac arrest. Currently known tests for such allergies cannot be performed at the patient bedside as they are time consuming, and involve numerous steps and equipment.

US 2004/0116830 discloses a blood testing device comprising: an intermediate portion between a proximal end and a distal end; a pressure compensation system to regulate the pressure in the intermediate portion; and a distal end cap comprising a first opening configured to receive a test strip. The intermediate portion has one or more than one opening at its proximal end and comprises a hollow, central chamber surrounded by an outer wall. The one or more than one opening may comprise a flow valve.

US 2008/0255473 describes a fluid drawing device and a fluid sampling device. The fluid sampling device includes a user depressible diaphragm that can expel air out of a vacuum chamber. On release the diaphragm returns to its original position so as to draw sample fluid in through a sample port.

It is therefore desirable to provide a device for integrating allergy testing into a medical device.

### Summary of the disclosure

The disclosure provides an allergy detection system for use during catheterization. The allergy detection system is incorporated into specialized syringes, connectors for use with standard syringes, or can be an independent test module designed for the sole purpose of allergy detection. The detection system features a test strip, such as an immunochromatographic test strip, and a structure to couple the system to a connector, syringe, or a housing, to form an independent test module. The detection system, by way of test strips, is used to detect potential allergic reactions.

According to an aspect of the invention there is provided a device for testing the presence or absence of a substance in blood, comprising: a coupler or hub that is capable of coupling with a needle, wherein the needle is for withdrawing blood from a subject and for introducing the withdrawn blood into the device; a first chamber for receiving the withdrawn blood; a second chamber for receiving at least some of the withdrawn blood from the first chamber; a test strip held within the second chamber; a wall or panel that separates the first chamber from the second chamber; and at least one aperture in the wall or panel configuring for allowing blood to pass from the first chamber to the second chamber. The test strip is capable of producing a visible signal that indicates the presence or absence of the substance in the withdrawn blood, and the second chamber comprises a window for viewing the test strip, the window allowing visual detection of the visible signal. The second chamber comprises a flexible dome, wherein the flexible dome is capable of flexing radially outward to create a partial vacuum that draws blood out of the first chamber and into the second chamber and of flexing radially downwards to increase the pressure within the second chamber. A sliding latch is operatively linked to the flexible dome and is capable of: being slid over the flexible dome to compress the flexible dome radially downwards and increase the pressure within the second chamber, and being slid away from the flexible dome to release the flexible dome and allow the flexible dome to flex radially outwards to create the partial vacuum in the second chamber.

According to another aspect of the invention there is provided a system comprising, in combination, a medical instrument that is capable of holding blood, and a medical device that comprises: (a) a coupler or hub that is capable of coupling with a needle, wherein the needle is for withdrawing blood from a subject and for introducing the withdrawn blood into the device; (b) a first chamber for receiving the withdrawn blood; (c) a second chamber configured to receive at least some of the withdrawn blood from the first chamber; (d) a test strip held within the second chamber; (e) a wall or panel that separates the first chamber from the second chamber; and (f) at least one aperture in the wall or panel configuring for allowing blood to pass from the first chamber to the second chamber; (g) wherein the test strip produces a visible signal that indicates the presence or absence of the substance in the withdrawn blood; (h) wherein the second chamber comprises a window for viewing the test strip, the window allowing visual detection of the visible signal. The medical device further comprises: (i) the second chamber comprises a flexible dome capable of flexing radially outward to create a partial vacuum that draws blood out of the first chamber and into the second chamber, and of flexing radially downwards to increase the pressure within the second chamber; (j) a sliding latch is operatively linked to the flexible dome and is capable of: being slid over the flexible dome to compress the flexible dome radially downwards and increase the pressure within the second chamber, and being slid away from the flexible dome to release the flexible dome and allow the flexible dome to flex radially outwards to create the partial vacuum in the second chamber.

A selection of optional features is set out in the dependent claims.

There has thus been outlined, rather broadly, certain embodiments of the invention in order that the detailed description thereof, herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present disclosure. It is important, therefore, that the claims be regarded as including such equivalent constructions insofar as they do not depart from the scope of the present disclosure.

Components for the methods and devices of the disclosure are available, for example, from any major medical device company, for example, Medtronic of Minneapolis, MN; Advanced Cardiovascular Systems in Santa Clara, CA; Baxter International of Deerfield, IL; Abbott Laboratories at Abbott Park, IL, Edwards Lifesciences, Irvine, CA, and Boston Scientific of Natick, MA. Components of the present disclosure can be made, without limitation, by molding, blow molding, slush molding, injection molding, rotational molding, compression molding, extrusion, thermoforming, stamping, calendaring, and so on (Brazel, CS; Rosen, SL (2012) Fundamental Principles of Polymeric Materials. Wiley, Hoboken, NJ).

### Brief descriptions of the drawings

FIG. 1 illustrates a test strip in accordance with an embodiment of the invention.
FIGS. 2 and 3 illustrate a perspective view of an independent allergy testing module in accordance with an embodiment of the invention.
FIGS. 4 and 5 illustrate a sectional view of the independent allergy testing module illustrated in FIGS. 2 and 3 in accordance with an embodiment of the invention.
FIG. 6 illustrates an exploded view of the independent allergy testing module illustrated in FIGS. 2-5 in accordance with an embodiment of the invention.
FIG. 7 illustrates a syringe with integrated allergy testing in accordance with an embodiment of the invention.
FIG. 8 illustrates a sectional view of the syringe with integrated allergy testing illustrated in FIG. 7 in accordance with an embodiment of the invention.
FIG. 9 illustrates an exploded view of the syringe with integrated allergy testing illustrated in FIGS. 7 and 8 in accordance with an embodiment of the invention
FIG. 10 illustrates a connector with integrated allergy testing in accordance with an embodiment of the invention.
FIG. 11 illustrates a sectional view of the connector with integrated allergy testing illustrated in FIG. 10 in accordance with an embodiment of the invention.
FIG. 12 illustrates an exploded view of the connector with integrated allergy testing illustrated in FIGS. 10 and 11 in accordance with an embodiment of the invention.
FIG. 13 (A, B) illustrates the cap that is a part of the module embodiment, and structures in the vicinity of the cap.

### Detailed description

The present disclosure provides in some embodiments, an allergy detection system for integration into medical devices. For instance, in accordance with embodiments of this invention, the allergy detection system can be incorporated into a specialized syringe, a connector for use with a standard syringe, or an independent test module, designed for the sole purpose of allergy detection. The detection system can include, without limitation, an immunochromatographic membrane and structure to couple it to a connector or syringe or a housing to form an independent test module. The detection system can be used to detect any potential allergies that can be identified using immunochromatography.

The present disclosure provides a device and related methods for point of care testing. For example, where a needle is introduced for use in the Seldinger technique, the needle can be used in conjunction with device and method of the present disclosure for detecting antibodies and predicting adverse allergic reactions. The *Seldinger* technique, includes the initial step of inserting a needle into a patient's blood vessel, where the Seldinger technique concludes with replacing the needle with a catheter or other medical device. The end-result is that the catheter or other medical device is situated in a subject's blood vessel or other body cavity. The *Seldinger* technique, and variations thereof, and devices used to perform this technique, are described in Seldinger (1953) Acta Radiologica 39:368-376; U.S. Pat. No. 7,722,567 issued to Tal, 7,972,307 issued to Kraus, et al, and 7,938,806 issued to Fisher, et al. Following testing by device and method of the present disclosure, and where the test gives a negative result (indicating that the subject is not allergic), the clinician can proceed with the *Seldinger* technique, and replace the needle with a catheter that contains the antigenic drug, antigenic antiseptic, antigenic adhesive, or antigenic latex. Alternatively, if the test gives a positive result and indicates that the subject is likely to be allergic to the antigenic drug, antigenic antimicrobial agent, antigenic antiseptic, antigenic adhesive, or antigenic latex, then the clinician refrains from using the medical device. Where the test gives a positive result, the clinician can use an alternative medical device that does not contain the offending drug, antiseptic, adhesive, or latex, and the like.

In this way, the device and procedure of the present disclosure is used at the point of care, and is integrated into the procedure for using any medical device of interest, and where the device and procedure of the present disclosure can give the clinician a yes/no answer. The device of the present disclosure can be a physical part of a device used for the medical procedure of interest, for example, the device can be connected to a syringe that is used in the *Seldinger* technique or other procedure. This connection can be by way of a luer lock, Storz lock, or other coupler. Alternatively, the device of the present procedure can be integrated into a syringe, that is permanently molded into the syringe, that is, used in the *Seldinger* technique or other procedure

In exclusionary embodiments, the present disclosure includes only test-strip devices that are integrated into a syringe. In another exclusionary embodiment, the present disclosure excludes test-strip devices that are not integrated into a syringe. In exclusionary embodiments, the present disclosure includes only test-strip devices that are integrated into a needle hub, trocar, dilator, syringe, syringe barrel, introducer, and the like. In another exclusionary embodiment, the present disclosure excludes test-strip devices that are not integrated into a needle hub, that are not integrated into a trocar, that are not integrated into a dilator, that are not integrated into a syringe or syringe barrel, that are not integrated into an introducer, and the like.

### Immune disorders arising from exposure to medical devices and pharmaceuticals

The present disclosure provides a device and related methods, for detecting antibodies against components of medical devices or components of pharmaceuticals, and for quantitating or for predicting allergic reactions in a subject to be exposed to the medical device or the pharmaceutical. What can be quantitated is, for example antibodies of one or more of the classes IgG, IgA, IgM, IgD, or IgE, that are present in a subject's bloodstream or in any biological fluid. The device and related methods are configured for quantifying or for predicting anaphylactic-type reactions or bronchospasm (Layton et al (1989) Clin. Exp. Allergy. 19:307-314; Terazawa et al(1998) Anesthesiology. 89:1298-1300), allergic contact dermatitis, irritant contact dermatitis, photoallergic contact dermatitis, or immediate type contact reactions (Kutzscher (2012) Clin. J. Oncol. Nurs. 16:E48-55; Krasteva (1999) Eur. J. Dermatol. 9:144-159), arising from exposure to an antimicrobial agent or an antiseptic, e.g., a biguanide antiseptic.

### Allergic reactions against and antibodies to antimicrobial agents, and to other components of medical devices

Device and methods of the present disclosure can detect antibodies, present in a subject's blood, serum, or other biological fluid, where the antibodies specifically bind to an antimicrobial agent, such as an antiseptic or antibiotic (Torres et al (2009) 19:67-68), antibiotics such as rifampicin (Feng et al (2011) Eur. J. Dermatol. 21:696-699), adhesives on catheters (Meikle et al (2012) Can. J. Anaesth. 59:815-816), thimerosol or other preservatives in medical devices (Ancona et al (1990) Dermatol. Clin. 8:95-105). Allergic reactions to latex, and antibodies against latex, have been described (Patriarca et al (2002) J. Investig. Allergol. Clin. Immunol. 12:169-176; Unsel et al (2012) lnt. Arch. Allergy Immunol. 158:281-287). Allergic reactions (analphylaxis) can occur in response to medical devices impregnated with one or more antiseptics (Darouiche and Raad (1999) New Engl. J. Med. 1762 (1 page)). Antibodies of the classes IgG and IgA are associated with drug-induced eruptions (pemphigus folicaeus) (Feng et al (2011) Eur. J. Dermatol. 21:696-699). Antibodies of the classes IgG and IgE are associated with allergic reactions to antiseptics (Layton et al (1987) Mol. Immunol. 24:133-141). Antibodies of the IgE class have been identified that are responsible for hypersensitivity to various beta-lactam antibiotics (Gunez et al (2012) J. Investig. Allergol. Clin. Immunol. 22:41-47). IgE antibodies have also been identified that specifically react against latex (Galindo et al (2011) J. Investig. Allergol. Clin. Immunol. 21:459-465).

The term "antimicrobial" encompasses, but is not limited to, antiseptics and antibiotics.

### Detecting antibodies and other molecules that can provoke adverse events

Antibodies in blood or in other biological fluids can be detected, for example, by way of reagents in a test strip. The reagents can be covalently or non-covalently attached to the surface of a test strip, the reagents can be adsorbed to a test strip, the reagents can be absorbed to a test strip, the reagents can be covalently or non-covalently bound to interior of test strip, such as porous interior or fibrous interior, or any combination of the above. Reagents, test strips, substrates, for immune assays, including color reactions, and the like, are provided, for example, from US 7,544,324 issued to Tung et al, US 7,438,852 issued to Tung et al, US 7,989,217 issued to Yee et al, 7,910,381 of Ford et al. The present disclosure encompasses detection of antibodies, such as IgG, IgA, IgM, IgD, or IgE, detecting B cells that express IgE or other classes of antibodies, by way of test strip technology. Several antibody classes can be simultaneously expressed in the body, and that recognize the same antigen. Accordingly, the present disclosure provides a test strip that can detect antibodies two or more of the classes IgG, IgA, IgM, IgA, IgD, or IgE. In one embodiment, the test strip detects two different classes, and provides two different colors. In another embodiment, the test strip detects two different classes, and provides the same color for both of the respective color reactions, but at different locations in the test strip. In yet another embodiment, the test strip detects a plurality of different antibody classes (or detects a plurality of different specific antibodies that bind to respective different antigens, where all of these specific antibodies are in one particular class). Combinations of the previous embodiments are also provided, e.g., detecting IgG that binds to an antibiotic, and detecting IgE that binds to latex, or detecting an antibody that binds to an antimicrobial and an antibody that binds to a pathogen. Color reaction can be provided by a label that comprises a metal, a colloidal particle such as colloidal gold, a dye particle, or results from a reaction catalyzed by peroxidase or alkaline phosphatase (see US 7,262019 issued to Kovalenko, US 5,200,312 issued to Oprandy, US 5,637,468 issued to Mason, and US 6,503,726 issued to Anne). Where a gold labeled anti-IgE antibody is used, for example, a test sample (e.g., blood) reacts with the gold labeled anti-IgE antibody, forming a complex. The complex travels across a membrane where immobilized allergens capture the complex, resulting in a colored line (see US 7,629,127 issued to Hubscher).

The following provides a non-limiting example. For detection of anti-antiseptic antibodies, blood can be deposited at a sample receiving area located at a first end of the test strip. Flow of solvent from the first end of the test strip towards the opposite end of the test strip draws any antibodies towards the test strip, and draws the antibodies over a region of the test strip that contains conjugates of gold-antiseptic. When the antibodies migrate over the area that contains gold-antiseptic, the result is an insoluble complex that forms a visually detectable line. Conjugates of gold-antiseptic that are not trapped as part of this insoluble complex continue to migrate on the test strip, and do not contribute to the visual signal of the line. As an alternative to using a conjugate of gold-antiseptic, what can be used is a conjugate of gold with an antibody that specifically binds to anti-antiseptic antibodies (anti-idiotype antibodies). The above narrative applies to anti-antiseptic antibodies, but also to embodiments where the antigen is an antibiotic, a pharmaceutical, a drug, latex, an adhesive, and the like.

In a non-limiting embodiment, what is provided is a device that encompasses an immunoassay with all reagents embedded on a test strip that performs without the use of instruments. Total IgE in a biological fluid is bound by anti-human IgE immobilized on test strips in the IgE reactive field of the test strip. Visualization of captured IgE is indicated by colloidal gold labeled anti-human IgE. Excess labeled antibodies are captured by antibodies in the control field. A control line appears in the control field and determines when enough of the biological fluid has been collected. The presence of IgE in the sample is visualized by the appearance of a dark-colored line, e.g., a red line, in the reactive field and the control field. Absence of a line in the control field, regardless of the presence of line in the reactive field, indicates an invalid assay result. The area on the support identifying the reactive field and control field are on the back surface of the support in two colors. A sample with IgE in the normal reference range lacks a line in the reactive field but has a line in the control field. The presence of lines in the reactive and control fields indicates a sample with elevated total IgE levels. The device provides a qualitative/semi-quantitative measure of total IgE in the biological fluid when the intensity of color on the test strip is visually observed. The skilled artisan can divide or classify the different intensities of signal as negative or as positive, and the skilled artisan can prepare a standard operating procedure (SOP) that sets forth these different intensities. What is also provided is the above embodiment, but where it applies to other antibody classes, e.g., IgG, IgA, IgM, or IgD.

In a non-limiting embodiment, what is provided is a device that comprises a series of capillary beds (elements), for example, made of cellulose. The first element (a sample pad) acts as a sponge and can hold an excessive amount of biological fluid. Then, the fluid migrates to the second element (conjugate pad). The second element is manufactured with a conjugate, a dried format of particles in a soluble matrix that contains reagents that promote the desired chemical reaction between the target molecule (antigen) and its binding partner (antibody) that has been immobilized on the surface of the particle. At the time that the sample fluid dissolves the soluble matrix, it also dissolves the particles and the sample and conjugate mix while flowing through the porous structure. In this way, the analyte binds to the particles while migrating further through the third capillary bed. This material has one or more regions or stripes where a third molecule has been immobilized. By the time the sample-conjugate mix has migrated to these stripes, analyte has been bound on the particle and the third capture molecule binds the complex. When additional fluid passes the stripes, particles accumulate and the stripe-area changes color. In embodiments, there can be two stripes: one (the control) that captures any particle and demonstrates that reaction is in working order, the second contains a specific capture molecule and only captures those particles on which an analyte molecule has been immobilized.

### Duration of detection

In embodiments, what is provided is a device that is capable of detecting an antibody responsible for an allergic reaction, for example, to an antibiotic or antiseptic,
(1) During a blood draw that is dedicated to this detection;
(2) Only during a blood draw that is dedicated to this detection, with no capability during subsequent procedures, e.g., during insertion of a catheter or other medical instrument. This is an exclusionary embodiment;
(3) During a period of time when a catheter or other medical instrument is indwelling in a subject, that is, when a catheter or other medical instrument is operably linked to a subject. This period of time can be, e.g., at least ten minutes, at least one hour, at least one day, at least one week, at least one month, and the like;
(4) Only during a period of time when a catheter or other medical instrument is indwelling in a subject, but not capable of detecting the antibody during a blood draw that is solely dedicated to the detection of the antibody. This is an exclusionary embodiment.

### Antibodies and other reagents

"Specifically" or "selectively" binds, when referring to a ligand/receptor, nucleic acid/complementary nucleic acid, antibody/antigen, or other binding pair (e.g., a cytokine to a cytokine receptor) indicates a binding reaction which is determinative of the presence of the protein in a heterogeneous population of proteins and other biologics. Thus, under designated conditions, a specified ligand binds to a particular receptor and does not bind in a significant amount to other proteins present in the sample. Specific binding can also mean, e.g., that the binding compound, nucleic acid ligand, antibody, or binding composition derived from the antigen-binding site of an antibody, of the contemplated method binds to its target with an affinity that is often at least 25% greater, more often at least 50% greater, most often at least 100% (2-fold) greater, conventionally at least ten times greater, more normally at least 20-times greater, more typically at least 100-times greater, and most typically at least 1000-fold than the affinity with any other binding compound (see, e.g., US 2012/0121643 of Dubensky, which is incorporated by reference).

In a typical embodiment an antibody will have an affinity that is greater than about 10⁹ liters/mol, as determined, e.g., by Scatchard analysis (Munsen, et al. (1980) Analyt. Biochem. 107:220-239). It is recognized by the skilled artisan that some binding compounds can specifically bind to more than one target, e.g., an antibody specifically binds to its antigen, binds to lectins by way of the antibody's oligosaccharide, and also binds to an Fc receptor by way of the antibody's Fc region.

In embodiments, the disclosure provides test strip that is capable of detecting antibodies in blood, where the antibodies specifically bind to a pathogen. Anti-pathogen antibodies have been characterized. Reagents and kits are available for detecting antibodies that specifically bind to viruses (e.g., HIV, HCV,HBV), fungi, and bacteria (see, e.g., Novack et al (2006) J. Clin. Microbiol. 44:2909-2913; Hennessey et al (2009) J. Urban Health. 86:93-105; Thurman et al (2009) Clin. Infect. Dis. 48:1244-1249; Alexander et al (1996) J. Clin. Microbiol. 34:1180-1183).

"Sample" refers to a sample from a human, animal, placebo, or research sample, e.g., a cell, tissue, organ, fluid, gas, aerosol, slurry, colloid, or coagulated material. The "sample" may be tested in vivo, e.g., without removal from the human or animal, or it may be tested in vitro. The sample may be tested after processing, e.g., by histological methods. "Sample" also refers, e.g., to a cell comprising a fluid or tissue sample or a cell separated from a fluid or tissue sample. "Sample" may also refer to a cell, tissue, organ, or fluid that is freshly taken from a human or animal, or to a cell, tissue, organ, or fluid that is processed or stored.

A composition that is "labeled" is detectable, either directly or indirectly, by spectroscopic, photochemical, biochemical, immunochemical, isotopic, or chemical methods. For example, useful labels include ³²P, ³³P, ³⁵S,¹⁴C, ³H, ¹²⁵I, stable isotopes, epitope tags fluorescent dyes, electron-dense reagents, substrates, or enzymes, e.g., as used in enzyme-linked immunoassays, or fluorettes (see, e.g., Rozinov and Nolan (1998) Chem. Biol. 5:713-728).

### Detailed descriptions of the figures

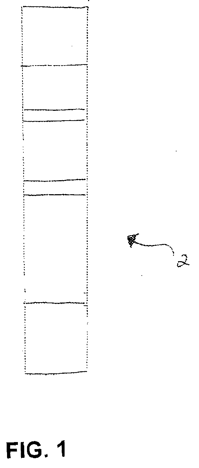

The invention will now be described with reference to the drawing figures, in which like reference numerals refer to like parts throughout. As illustrated in FIG. 1, the allergy detection system can include an immunochromatographic membrane 2 (PVDF or nitrocellulose material) in strip or cassette format that can detect elevated levels of an allergen by employing a highly sensitive detecting antibody and a carrier-protein conjugated hapten. The antibody is labeled with a signal generator (e.g., colloidal gold), which is placed in the dry state at a predetermined site on the membrane. Using this novel approach the clinician (catheter inserters) would be able to determine if a patient is at risk of developing an allergic reaction before potentially introducing an allergen into a patient's system. The system also provides ease of use to the hospital staff as it does not require any additional step, equipment or time other than what is needed for standard procedures.

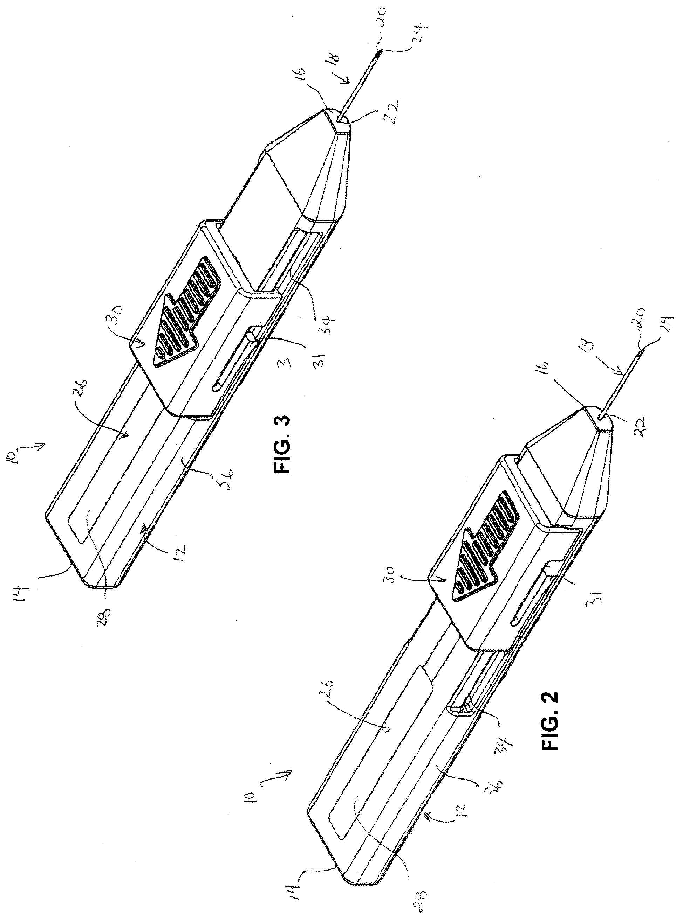

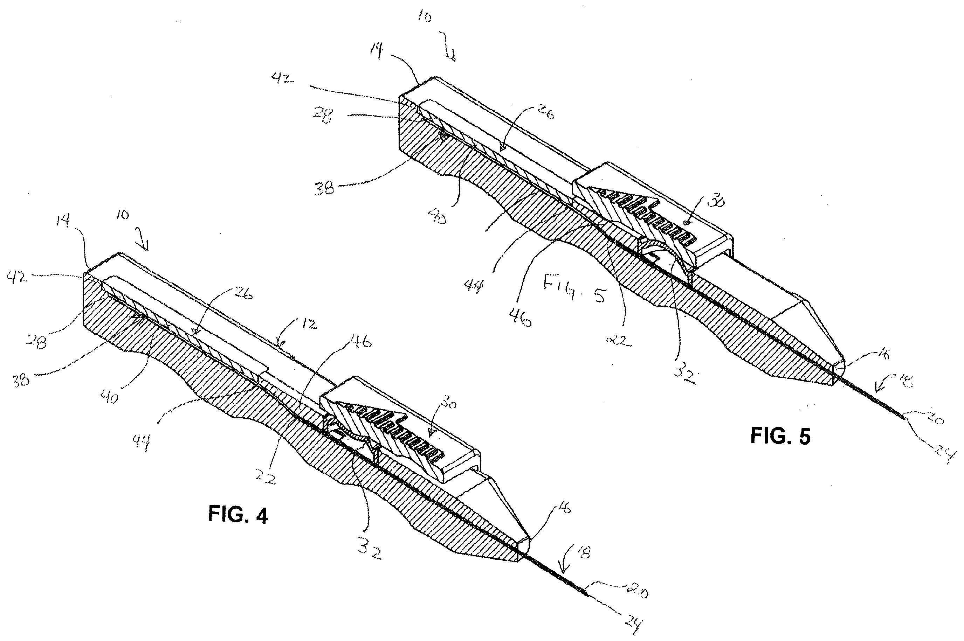

FIGS. 2 and 3 illustrate an independent testing module in accordance with an embodiment of this invention. FIG. 2 illustrates the independent testing module 10 in its inactive state and FIG. 3 illustrates the independent testing module 10 in its active state. The independent testing module 10 includes a housing 12 having a proximal end 14 and a distal end 16. A needle 18 can extend from the distal end 16 of the housing 12.
The needle 18 can include a sharpened distal end 20 and a proximal end 22 that extends into the housing 12. The needle 18 can be generally configured as a hypodermic-type needle with a bore 24 extending through its length. The testing module 10 can also include a viewing window 26, through which test strip 28 can be observed. The test strip 28 is disposed within the housing 12 behind the window 26, such that the operator can view any changes in the strip 28 during use. The testing module 10 can also include a slide 30 and a vacuum button 32 (as illustrated in FIGS. 4-6). In the inactive state illustrated in FIG. 2, the slide 30 is positioned toward the distal end of the testing module 10. When the needle 18 is inserted into the patient and testing is required the slide 30 can be moved from its distal, forward position to a proximal position. Slot 34, positioned on a side 36 of housing 12 in FIGS. 2 and 3, is configured to lock the slide 30 into place after it has been activated into the proximal position.

The button can comprise an elastic material, for example, silicone, latex, or polyurethane. In one non-limiting embodiment, button is thinner at the peak of the button's dome, and is thicker at the base of the dome.

FIGS. 4 and 5 illustrate a sectional view of the independent testing module 10 illustrated in FIGS. 2 and 3. FIG. 4 is a sectional view of the independent testing module 10 in the inactive position, as illustrated in FIG. 2, and FIG. 5 is a sectional view of the independent testing module 10 in the active position, as illustrated in FIG. 3. The housing 12 can define an inner space 38 near the proximal end 14 of the testing module 10. The test strip 28 can be disposed in this inner space 38. In addition, an absorbent pad 40 can be integrated into the bottom of the test strip 28 in this inner space 38. The test strip 28 can include a proximal end 42 and a distal end 44. Cap 46 is positioned on top of the junction between the proximal end of the needle 22 and the distal end 44 of the test strip 28.

The following concerns non-limiting embodiments of cap (46). The cap holds or positions the test strip. The cap holds or positions the proximal end of the needle. Also, the cap creates or defines small vacuum pathways from the vacuum button to the proximal end of the needle. In a preferred embodiment, the cap (46), is not removable.

In a non-limiting embodiment of the present disclosure, the absorbent pad is an integrated part of the test strip. In another non-limiting embodiment, the absorbent pad is separate from, and does not occupy the same space as, the test strip. The absorbent pad increases the total volume of sample that enters the test strip. The absorbent pad can block the migration of blood cells, and thereby provide a clean field for development and detection (by clinician's eyes) of the color reaction. In addition, or alternatively, the absorbent pad can draw the biological fluid by way of capillary attraction, resulting in wetting of a broad area of the test strip. In another embodiment, the device comprises only a test strip and does not contain any absorbent pad. In one embodiment that does not contain an absorbent pad, the test strip does not disperse fluid by way of capillary attraction, and the biological fluid is sufficiently dispersed without capillary attraction. In another embodiment, the test strip is sufficiently thick (or thin but sufficiently opaque) that it blocks passage of red blood cells to the viewable area where the color reaction takes place. In yet another embodiment, the test strip has capillary attraction and is also sufficiently thick (or thin but sufficiently opaque) to block passage of red blood cells, and to prevent the red blood cells from residing in the viewable area where the color reaction takes place). Absorbent pad can comprise cellulose, polyvinylidene fluoride (PVDF), nitrocellulose, and the like. Filter material suitable as a matrix for test strips, and for serving as a reaction area for color reagents, and optionally for blocking red blood cells, and optionally for serving as a capillary attractant for attracting and dispersing a biological fluid, are available (EMD Millipore, Darmstadt, Germany; Millipore, Billerica, MA; Membrane Solutions, Plano, TX). US 4,855,240 of Rosenstein, US 4,703,017 of Campbell, and US 4,376,110 of David et al, disclose absorbent materials, capillary materials, test strips, labels, and immunoassays.

FIGS. 4 and 5 also illustrate a vacuum button 32 of the independent testing module 10. In the inactivated position illustrated in FIG. 4, the vacuum button 32 is depressed by slide 30. This keeps the vacuum button 32 depressed until the needle 18 is inserted and ready to extract blood. As illustrated in FIG. 5, once the device is in position, the slide 30 is retracted to the active position, releasing vacuum button 32, thus creating a vacuum and allowing blood to flow into the bore 24 of the needle 18. Locking the slide 30 in the active position prevents depression of the vacuum button 32, when the needle 18 is inserted into a vein of the patient, in turn preventing air from potentially being pushed into the access point or blood being squirted from the device after it has been used. The blood aspirated into the testing module 10 is absorbed from the bottom end of the testing strip 28. The absorbent pad 40 can absorb the plasma sample after the completion of the signal detection reaction. The absorbent pad 40 can also include a porous backing and an absorbent dispersed, adsorbed, or coated into the pores of the porous backing. A color signal can appear within approximately 10 minutes within the viewing window 26, but could take less time or more time depending on the detecting antibodies contained in the test strip.

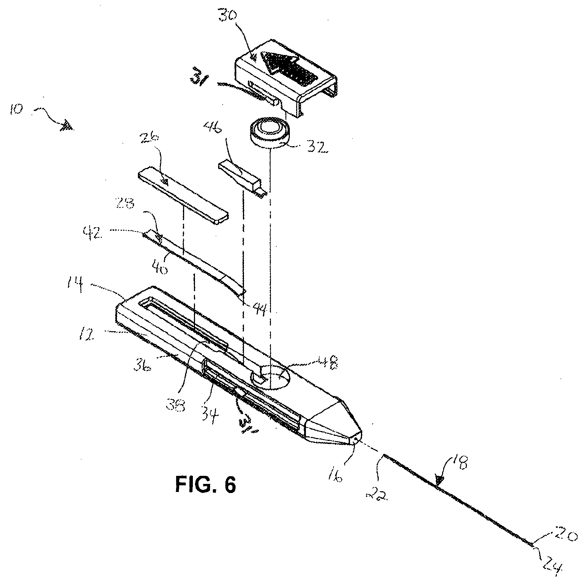

FIG. 6 illustrates an exploded view of the independent testing module 10 as shown in FIGS. 2-5. As shown in FIG. 6, the testing module includes the housing 12 and a needle 18 coupled to a distal end 16 of the housing 12. The housing 12 also defines the inner space 38 near the proximal end 14 of the testing module 10. The test strip 28 can be disposed in this inner space 38. In addition, an absorbent pad 40 can be integrated into the test strip 28 in this inner space 38. The test strip 28 can include a proximal end 42 and a distal end 44. Cap 46 is positioned on top of the junction between the proximal end of the needle 22 and the distal end 44 of the absorbent pad 40. FIG. 6 also illustrates the slide 30 and the vacuum button 32 which work together to release the vacuum for the independent testing module such that it can aspirate the blood sample. The slide 30 and vacuum button 32 also prevent the vacuum button from being re-depressed to prevent pushing any air into the entry site or allowing any blood to spurt out after use. The vacuum button 32 sits within an opening 48 defined by the housing 12. The slide 30 can lock into position via the slot 34, also illustrated in FIGS. 2 and 3. As illustrated, tab 31 interacts with a notch 31' in the architecture of slot 34 in order to lock the slide 30 into place. However, any suitable means of locking the slide into place can be used. Locking the slide 30 in the active position prevents depression of the vacuum button 32, when the needle 18 is inserted into a vein of the patient, in turn preventing air from potentially being pushed into the access point or blood being squirted from the device after it has been used. Cap 46 is also shown and is placed over the junction between the testing strip 28 and the proximal end 22 of the needle 18.

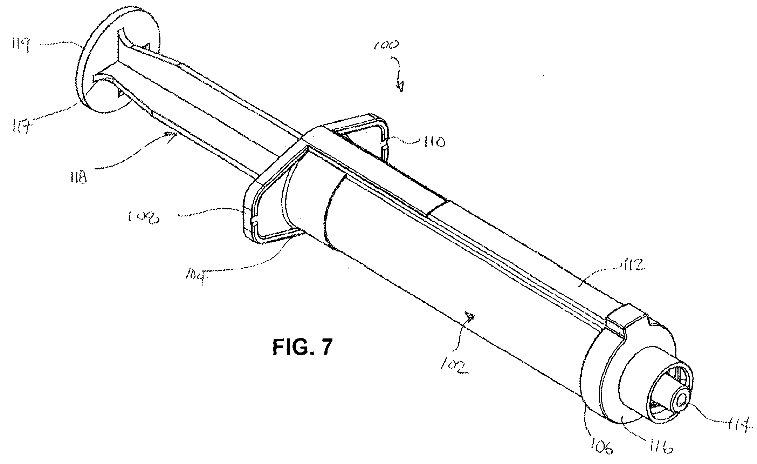

FIG. 7 illustrates a syringe with integrated allergy testing, in accordance with another embodiment of the integrated allergy testing system. As shown in FIG. 7, the syringe 100 includes a housing 102 having a proximal end 104 and a distal end 106. The proximal end 104 of the housing 102 can include flanges 108, 110 which can serve as finger rests during use of the syringe 100. The housing 102 can include a window 112 positioned such that test strip positioned below the window is visible to a user of the syringe 100. The test strip resides within its own lumen or channel, such that only the distal end of the test strip is exposed to the blood that is aspirated within the syringe housing 102. A needle hub 114 can be positioned at the distal end 106 of the housing 102 and can be configured to couple to a hypodermic-type needle for aspirating blood into a lumen of the syringe 100. A syringe housing cap 116 can be coupled to the distal end 106 to hold the test strip in the housing 102. The syringe 100 can also include a plunger 118 extending into the lumen of the housing 102 for creating negative pressure to aspirate blood into the lumen of the syringe. The plunger 118 can include a thumb rest 119 positioned at a proximal end 117 of the plunger 118.

In embodiments, where test strip resides in its own lumen or channel, blood or other biological sample enters channel through a hole, slit, passage, or crevice, that is about 5% the distal-to-proximal length of the channel, about 10% the distal-to-proximal length of the channel, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, or longer, than the distal-to-proximal length of the channel. Where the slit or crevice is about 100% the distal-to-proximal length of the channel, the result is that blood entering the syringe, or entering a corresponding structure in the module (10), contacts that entire test strip essentially simultaneously.

Regarding the orientation of the channel with respect to the hole, slit, passage, or crevice, the hole, slit, or crevice, can start at a point corresponding to the distal-most portion of the channel, and then migrate proximally. Alternatively, the hole, slit, or crevice, can start at a point corresponding to the proximal-most portion of the channel, and then migrate distally. In yet another alternative, the hole, slit, passage, or crevice, can be situated entirely between the distal-most and proximal-most portions of the channel. For example, the hole or passage can be situated near the half-way point of the channel, where blood entering the hole migrates both in the proximal and in the distal directions.

In embodiments where the hole, slit, passage, or crevice, is about 5% the distal-to-proximal length of the channel, it may be the case that only about 5% of the test strip is wetted by the blood or other biological sample. Alternatively, in embodiments where the hole, silt, passage, or crevice, is about 5% the distal-to-proximal length of the channel, it may be the case that greater than about 5% of the test strip is wetted by the blood or other biological sample, where this greater than amount results from migration of the biological sample through the test strip. Migration can by via diffusion, capillary force, or by a combination of diffusion and capillary force. The above narrative also applies to configurations where the hole, slit, passage, or crevice, is less than 90%, less than 80%, less than 70%, less than 60%, less than 50%, less than 40%, less than 30%, less than 20%, less than 10%, less than 5%, of the length of the distal-to-proximal length of the channel.

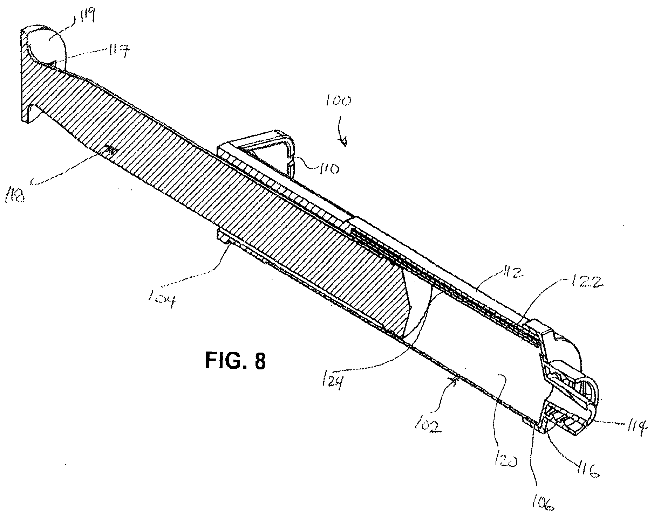

FIG. 8 illustrates a sectional view of the syringe illustrated in FIG. 7. As shown in FIG. 8, the syringe 100 includes the housing 102 defining a lumen 120, extending therethrough. The plunger 118 is shown extending into the lumen 120 of the housing 102. Additionally, FIG. 8 illustrates test strip 122 positioned within the housing 102 beneath window 112. The syringe housing cap 116 holds the test strip 122 in place beneath the window 112 for observation by the practitioner. Alternately, the test strip 122 can take the form of a cassette or any other appropriate form for positioning within the syringe 100. In addition, an absorbent pad 124 can be integrated into the test strip 122. The absorbent pad 124 can absorb the plasma sample after the completion of the signal detection reaction. The absorbent pad 124 can also include a porous backing and an absorbent dispersed, adsorbed, or coated into the pores of the porous backing. A color signal can appear within approximately 10 minutes within the viewing window 112, but could take less time or more time depending on the antibodies contained in the test strip.

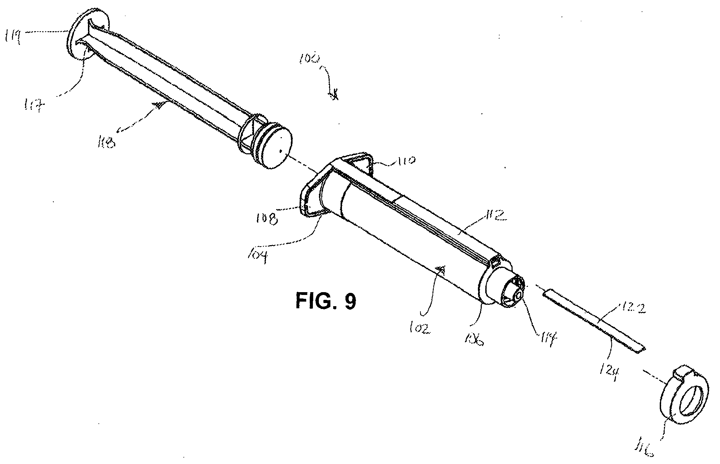

FIG. 9 illustrates an exploded view of the syringe illustrated in FIGS. 7 and 8. FIG. 9 shows the housing 102 having a window 112 for the test strip 122. The syringe housing cap 116 can be used to hold the test strip in place, such that it does not move during testing. The plunger 118 can be configured to slide within the lumen 120 of the housing 112.

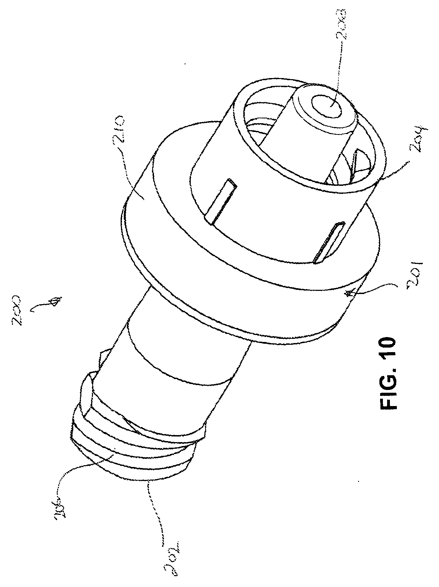

FIG. 10 illustrates a connector design for the integrated testing system in accordance with an embodiment of the invention. The connector 200, includes a housing 201, having a proximal end 202 and a distal end 204, and can be configured for placement between a syringe body and a needle for the syringe. The connector 200 can be connected to the syringe body in a variety of different ways such as frictional hold, tabs, or any other suitable coupling. As illustrated in FIG. 10, the connector 200 can be coupled to the syringe via luer threads 206 positioned at the proximal end 202 of the connector 200. The connector 200 can also include a coupling 208 for coupling the connector 200 to a needle. The needle can take the form of a hypodermic-type needle or any other needle suitable for aspirating blood through the connector 200. The connector also includes a window 210 through which test strip (not shown) can be visualized.

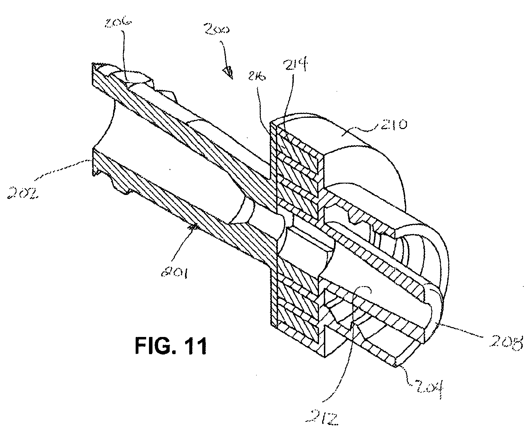

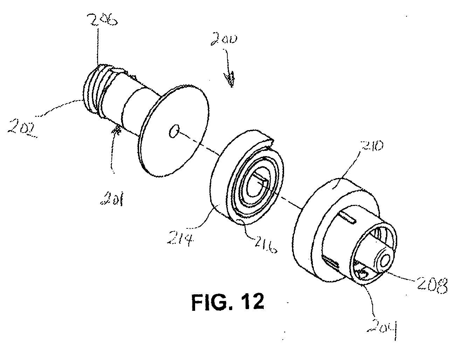

FIG. 11 illustrates a sectional view of the embodiment shown in FIG. 10 and FIG. 12 illustrates an exploded view of the embodiment illustrated in FIGS. 10 and 11. FIGS. 11 and 12 illustrate the test strip 214 disposed within the connector housing 201. As illustrated, the test strip 214 is spirally wound within the connector housing 201. The test strip 214 can be viewed through window 210 in the housing 201. Blood can be aspirated into a lumen 212 defined by housing 201, when testing is required. In addition, an absorbent pad 216 can be positioned beneath the test strip 214. The absorbent pad 216 integrated into the test strip 214 can absorb the plasma sample after the completion of the signal detection reaction. The absorbent pad 216 can also include a porous backing and an absorbent dispersed, adsorbed, or coated into the pores of the porous backing. A color signal can appear within approximately 10 minutes within the viewing window 210, but could take less time or more time depending on the antibodies contained in the blood sample.

Alternately test strip, for example, an immunochromatographic test strip, can be incorporated into a number of different medical devices. One such example is an test strip incorporated into a "hemo-hopper," such as a blood reservoir in a medical tray or kit. The blood aspirated during access to the vessel would be disposed into the hemo-hopper. The sample could then be absorbed into the test strip. In addition, an absorbent pad can be positioned beneath the test strip. The absorbent pad can absorb the plasma sample after the completion of the signal detection reaction. The absorbent pad can also include a porous backing and an absorbent dispersed, adsorbed, or coated into the pores of the porous backing. A color signal can appear within approximately 10 minutes, but could take less time or more time depending on the antibodies contained in the blood sample.

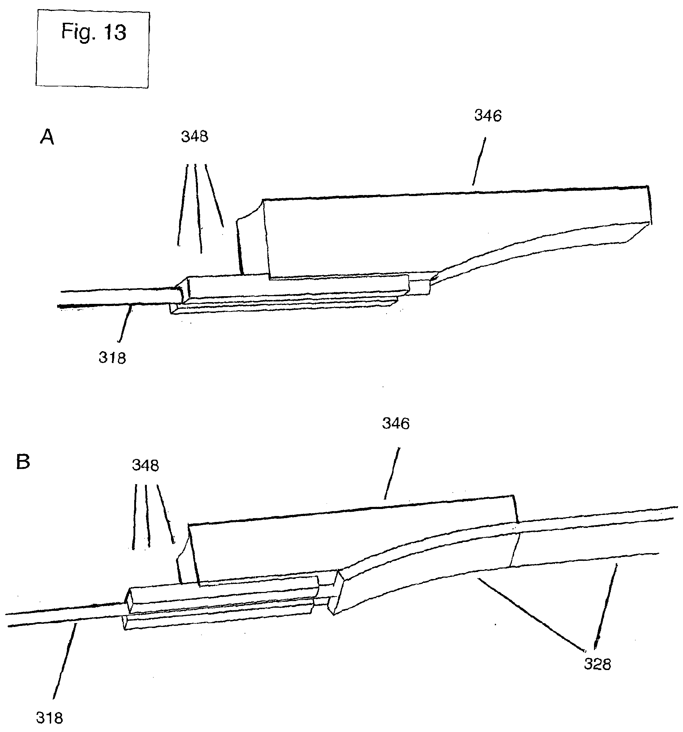

FIG. 13 (A, B) illustrates the end-cap that is a part of the module embodiment, and structures in the vicinity of the cap. The end-cap (346) is in contact with needle (318). The end-cap (346) defines part of cavity (348) where the vacuum button (32) can fit. A non-limiting embodiment of vacuum button (32) is shown in FIGS. 4-6. FIG. 13(A) shows cap without test strip, and FIG. 13 (B) shows cap with test strip.

While the method and apparatus have been described in terms of what are presently considered to be the most practical and preferred examples, it is to be understood that the disclosure need not be limited to the disclosed embodiments. It is intended to cover various modifications and similar arrangements included within the scope of the claims, the scope of which should be accorded the broadest interpretation so as to encompass all such modifications and similar structures. The present disclosure includes any and all embodiments of the following claims.

## Claims

1. A device for testing the presence or absence of a substance in blood, comprising:
a coupler or hub that is capable of coupling with a needle (18), wherein the needle (18) is for withdrawing blood from a subject and for introducing the withdrawn blood into the device;
a first chamber for receiving the withdrawn blood;
a second chamber for receiving at least some of the withdrawn blood from the first chamber;
a test strip (28) held within the second chamber;
a wall or panel that separates the first chamber from the second chamber; and
at least one aperture in the wall or panel configuring for allowing blood to pass from the first chamber to the second chamber,
wherein the test strip (28) is capable of producing a visible signal that indicates the presence or absence of the substance in the withdrawn blood, and
wherein the second chamber comprises a window (26) for viewing the test strip (28), the window (26) allowing visual detection of the visible signal, **characterised in that**:
the second chamber comprises a flexible dome (32), wherein the flexible dome (32) is capable of flexing radially outward to create a partial vacuum that draws blood out of the first chamber and into the second chamber and of flexing radially downwards to increase the pressure within the second chamber; and
a sliding latch (30) is operatively linked to the flexible dome (32) and is capable of:
being slid over the flexible dome (32) to compress the flexible dome (32) radially downwards and increase the pressure within the second chamber, and
being slid away from the flexible dome (32) to release the flexible dome (32) and allow the flexible dome (32) to flex radially outwards to create the partial vacuum in the second chamber.

2. The device of claim 1, that comprises a syringe, wherein the first chamber is defined by a housing or barrel of the syringe.

3. The device of claim 1, that is capable of being coupled to a syringe and to the needle.

4. The device of claim 1, wherein the substance is an antibody that binds to an antimicrobial agent.

5. The device of claim 1, wherein the substance is an antibody of the class IgG, IgA, IgM, or IgE.

6. The device of claim 1, wherein the test strip (28) is an immunochromatographic test strip.

7. A system comprising, in combination, a medical instrument that is capable of holding blood, and a medical device that comprises:
(a) a coupler or hub that is capable of coupling with a needle, wherein the needle is for withdrawing blood from a subject and for introducing the withdrawn blood into the device;
(b) a first chamber for receiving the withdrawn blood;
(c) a second chamber configured to receive at least some of the withdrawn blood from the first chamber;
(d) a test strip (28) held within the second chamber;
(e) a wall or panel that separates the first chamber from the second chamber; and
(f) at least one aperture in the wall or panel configuring for allowing blood to pass from the first chamber to the second chamber,
(g) wherein the test strip (28) produces a visible signal that indicates the presence or absence of the substance in the withdrawn blood;
(h) wherein the second chamber comprises a window (26) for viewing the test strip (28), the window (26) allowing visual detection of the visible signal; and
**characterised in that** the medical device further comprises:
(i) the second chamber comprises a flexible dome (32) capable of flexing radially outward to create a partial vacuum that draws blood out of the first chamber and into the second chamber, and of flexing radially downwards to increase the pressure within the second chamber;
(j) a sliding latch (30) is operatively linked to the flexible dome (32) and is capable of:
being slid over the flexible dome (32) to compress the flexible dome (32) radially downwards and increase the pressure within the second chamber, and
being slid away from the flexible dome (32) to release the flexible dome (32) and allow the flexible dome (32) to flex radially outwards to create the partial vacuum in the second chamber.

8. The system of claim 7, wherein the combination is a reversible combination,
wherein the device and the medical instrument are held in combination by one or more of a clip, snap, lock, or tab.

9. The system of claim 7, wherein the medical instrument comprises one or more of a syringe, trocar, catheter, introducer, sheath, hub, pump, or valve.

## Patentansprüche

1. Vorrichtung zum Prüfen des Vorhandenseins oder des Fehlens einer Substanz im Blut, umfassend:
einen Koppler oder Hub, der mit einer Nadel (18) verbunden werden kann, wobei die Nadel (18) zum Abnehmen von Blut eines Patienten ist und zum Einführen des abgenommenen Bluts in die Vorrichtung; eine erste Kammer zum Aufnehmen des abgenommenen Bluts;
eine zweite Kammer zum Aufnehmen von mindestens einem Teil des abgenommenen Bluts aus der ersten Kammer;
einen Teststreifen (28), der in der zweiten Kammer gehalten wird;
eine Wand oder ein Paneel, das die erste Kammer von der zweiten Kammer trennt; und mindestens eine Öffnung in der Wand oder in dem Paneel, die so ausgelegt ist, dass sie Blut von der ersten Kammer in die zweite Kammer fließen lässt,
wobei der Teststreifen (28) ein sichtbares Signal erzeugen kann, das das Vorhandensein oder das Fehlen der Substanz im abgenommenen Blut anzeigt, und
wobei die zweite Kammer ein Fenster (26) zum Betrachten des Teststreifens (28) umfasst, wobei das Fenster (26) die optische Erkennung des sichtbaren Signals ermöglicht, **dadurch gekennzeichnet, dass**:
die zweite Kammer eine flexible Kuppel (32) umfasst, wobei die flexible Kuppel (32) sich radial nach außen biegen kann, um ein Teilvakuum zu erzeugen, das Blut aus der ersten Kammer und in die zweite Kammer zieht, und sich radial nach unten biegen kann, um den Druck innerhalb der zweiten Kammer zu erhöhen; und
ein Gleitriegel (30) ist operativ mit der flexiblen Kuppel (32) verbunden und kann Folgendes tun: über die flexible Kuppel (32) gezogen werden, um die flexible Kuppel (32) radial nach unten zu pressen und den Druck innerhalb der zweiten Kammer zu erhöhen, und
von der flexiblen Kuppel (32) weggeschoben zu werden, um die flexible Kuppel (32) freizugeben, und der flexiblen Kuppel (32) zu ermöglichen, sich radial nach außen zu biegen, um das Teilvakuum in der zweiten Kammer zu erzeugen.

2. Vorrichtung nach Anspruch 1, die eine Spritze umfasst, wobei die erste Kammer durch ein Gehäuse oder einen Mantel der Spritze definiert ist.

3. Vorrichtung nach Anspruch 1, die an eine Spritze und an die Nadel angekoppelt werden kann.

4. Vorrichtung nach Anspruch 1, wobei die Substanz ein Antikörper ist, der sich an ein antimikrobielles Mittel bindet.

5. Vorrichtung nach Anspruch 1, wobei die Substanz ein Antikörper der Klasse IgG, IgA, IgM oder IgE ist.

6. Vorrichtung nach Anspruch 1, wobei der Teststreifen (28) ein immunochromatografischer Streifen ist.

7. System, das in Kombination ein medizinisches Instrument, das Blut enthalten kann, und eine medizinische Vorrichtung umfasst, die Folgendes umfasst:
(a) einen Koppler oder Hub, der sich mit einer Nadel verbinden kann, wobei die Nadel zum Abnehmen von Blut eines Patienten ist und zum Einleiten des abgenommenen Bluts in die Vorrichtung;
(b) eine erste Kammer zum Aufnehmen des abgenommenen Bluts;
(c) eine zweite Kammer, die zum Aufnehmen von mindestens einem Teil des abgenommenen Bluts aus der ersten Kammer ausgelegt ist;
(d) einen Teststreifen (28), der in der zweiten Kammer gehalten wird;
(e) eine Wand oder ein Paneel, das die erste Kammer von der zweiten Kammer trennt; und
(f) mindestens eine Öffnung in der Wand oder in dem Paneel, die so ausgelegt ist, dass sie Blut von der ersten Kammer in die zweite Kammer fließen lässt,
(g) wobei der Teststreifen (28) ein sichtbares Signal erzeugt, das das Vorhandensein oder das Fehlen der Substanz im abgenommenen Blut anzeigt;
(h) wobei die zweite Kammer ein Fenster (26) zum Betrachten des Teststreifens (28) umfasst, wobei das Fenster (26) die optische Erkennung des sichtbaren Signals ermöglicht; und **dadurch gekennzeichnet, dass** die medizinische Vorrichtung ferner umfasst:
(i) die zweite Kammer eine flexible Kuppel (32) umfasst, die sich radial nach außen biegen kann, um ein Teilvakuum zu erzeugen, das Blut aus der ersten Kammer in die zweite Kammer zieht, und sich radial nach unten biegen kann, um den Druck innerhalb der zweiten Kammer zu erhöhen;
(j) ein Gleitriegel (30) ist operativ mit der flexiblen Kuppel (32) verbunden und kann Folgendes tun:
über die flexible Kuppel (32) gezogen werden, um die flexible Kuppel (32) radial nach unten zu pressen und den Druck innerhalb der zweiten Kammer zu erhöhen, und
von der flexiblen Kuppel (32) weggeschoben zu werden, um die flexible Kuppel (32) freizugeben, und der flexiblen Kuppel (32) zu ermöglichen, sich radial nach außen zu biegen, um das Teilvakuum in der zweiten Kammer zu erzeugen.

8. System nach Anspruch 7, wobei die Kombination eine reversible Kombination ist, wobei die Vorrichtung und das medizinische Instrument von einem oder mehreren Elementen aus einem Clip, einer Schnappvorrichtung, Sperre oder Tab kombiniert gehalten werden.

9. System nach Anspruch 7, wobei das medizinische Instrument ein oder mehrere Elemente aus einer Spritze, einem Trokar, Katheter, Inserter, Hüllrohr, Hub, Pumpe oder Ventil umfasst.

## Revendications

1. Dispositif destiné à tester la présence ou l'absence d'une substance dans le sang, comprenant :
un coupleur ou une garde qui est capable de se coupler à une aiguille (18), dans lequel l'aiguille (18) est destinée à prélever du sang d'un sujet et à introduire le sang prélevé dans le dispositif ;
une première chambre pour recevoir le sang prélevé ;
une seconde chambre pour recevoir au moins une certaine partie du sang prélevé de la première chambre ;
une bandelette d'essai (28) maintenue dans la seconde chambre ;
une paroi ou un panneau qui sépare la première chambre de la seconde chambre ; et
au moins une ouverture dans la paroi ou le panneau configurée pour permettre au sang de passer de la première chambre à la seconde chambre ;
dans lequel la bandelette d'essai (28) est capable de produire un signal visible qui indique la présence ou l'absence de la substance dans le sang prélevé et
dans lequel la seconde chambre comprend une fenêtre (26) pour observer la bandelette d'essai (28), la fenêtre (26) permettant une détection visuelle du signal visible, **caractérisé en ce que** :
la seconde chambre comprend un dôme flexible (32), dans lequel le dôme flexible (32) est capable de fléchir radialement vers l'extérieur pour créer un vide partiel qui aspire le sang de la première chambre dans la seconde chambre et de fléchir radialement vers le bas pour augmenter la pression dans la seconde chambre ; et
un verrou glissant (30) est lié en service au dôme flexible (32) et est capable :
de glisser par-dessus le dôme flexible (32) pour comprimer le dôme flexible (32) radialement vers le bas et augmenter la pression dans la seconde chambre et
de s'écarter en glissant du dôme flexible (32) pour libérer le dôme flexible (32) et permettre au dôme flexible (32) de fléchir radialement vers l'extérieur pour créer le vide partiel dans la seconde chambre.

2. Dispositif selon la revendication 1, qui comprend une seringue, dans lequel la première chambre est définie par un logement ou un corps de la seringue.

3. Dispositif selon la revendication 1 qui est capable de se coupler à une seringue et à l'aiguille.

4. Dispositif selon la revendication 1, dans lequel la substance est un anticorps qui se lie à un agent antimicrobien.

5. Dispositif selon la revendication 1, dans lequel la substance est un anticorps de la classe IgG, IgA, IgM ou IgE.

6. Dispositif selon la revendication 1, dans lequel la bandelette d'essai (28) est une bandelette d'essai immunochromatographique.

7. Système comprenant en combinaison un instrument médical qui est capable de contenir du sang et un dispositif médical qui comprend :
(a) un coupleur ou une garde qui est capable de se coupler à une aiguille, dans lequel l'aiguille est destinée à prélever du sang d'un sujet et à introduire le sang prélevé dans le dispositif ;
(b) une première chambre pour recevoir le sang prélevé ;
(c) une seconde chambre pour recevoir au moins une certaine partie du sang prélevé de la première chambre ;
(d) une bandelette d'essai (28) maintenue dans la seconde chambre ;
(e) une paroi ou un panneau qui sépare la première chambre de la seconde chambre ; et
(f) au moins une ouverture dans la paroi ou le panneau configurée pour permettre au sang de passer de la première chambre à la seconde chambre ;
(g) dans lequel la bandelette d'essai (28) est capable de produire un signal visible qui indique la présence ou l'absence de la substance dans le sang prélevé et
(h) dans lequel la seconde chambre comprend une fenêtre (26) pour observer la bandelette d'essai (28), la fenêtre (26) permettant une détection visuelle du signal visible ; et
**caractérisé en ce que** le dispositif médical comprend les éléments suivants :
(i) la seconde chambre comprend un dôme flexible (32) qui est capable de fléchir radialement vers l'extérieur pour créer un vide partiel qui aspire le sang de la première chambre dans la seconde chambre et de fléchir radialement vers le bas pour augmenter la pression dans la seconde chambre ;
(j) un verrou glissant (30) est lié en service au dôme flexible (32) et est capable :
de glisser par-dessus le dôme flexible (32) pour comprimer le dôme flexible (32) radialement vers le bas et augmenter la pression dans la seconde chambre et
de s'écarter en glissant du dôme flexible (32) pour libérer le dôme flexible (32) et permettre au dôme flexible (32) de fléchir radialement vers l'extérieur pour créer le vide partiel dans la seconde chambre.

8. Système selon la revendication 7, dans lequel la combinaison est une combinaison réversible, dans lequel le dispositif et l'instrument médical sont maintenus en combinaison par un(e) ou plus d'une agrafe, d'un déclic, d'un verrou ou d'une languette.

9. Système selon la revendication 7, dans lequel l'instrument médical comprend un(e) ou plus d'une seringue, d'un trocart, d'un cathéter, d'un introducteur, d'une gaine, d'une garde, d'une pompe ou d'une soupape.
